# EUROPEAN PATENT APPLICATION

(11) **EP 2 740 802 A2**
(43) Date of publication of application: **11.06.2014**
(21) Application number: 12822084.5
(22) Date of filing: 26.07.2012
(51) Int. Cl.: C12Q 1/68, C12N 15/11, B82Y 15/00

(54) **METHOD FOR DETECTING NUCLEIC ACID USING INTERCALATOR-CONJUGATED METAL NANOPARTICLES**

(30) Priority: 05.08.2011 KR 20110078052
(71) Applicant: Intellectual Discovery Co., Ltd., Seoul 135-090 (KR)
(72) Inventor: CHUNG, Bong Hyun, Daejeon 305-806 (KR); KIM, Sang Gyu, Daejeon 305-806 (KR); JO, Hyeon Min, Daejeon 305-806 (KR)
(74) Representative: Ter Meer Steinmeister & Partner
(86) International application number: PCT/KR2012/005971
(87) International publication number: WO 2013/022203

(57) **Abstract**

The present invention relates to a method for detecting nucleic acid using intercalator-conjugated metal nanoparticles. More particularly, the present invention relates to a method which involves applying a sample containing a target nucleic acid to a DNA chip having a substrate with a DNA probe fixed thereon, reacting metal nanoparticles in which intercalators coupled with a double helix nucleic acid are conjugated, and reacting a metal enhancing solution to thereby amplify the sizes of the metal nanoparticles and detect the target nucleic acid using the unaided eye. The method for detecting nucleic acid according to the present invention uses intercalator-conjugated metal nanoparticles, and thus enables detection of nucleic acid using the unaided eye without any other equipment.; Therefore, compared with conventional detection methods, the present invention exhibits the effects of reducing analysis costs and the time needed for detection, and enables miniaturization of the size of an apparatus, thereby enabling field diagnosis in a livestock farm, home, or the like.

<110>    KOREA RESEARCH INSTITUTE OF BIOSCIENCE AND BIOTECHNOLOGY

<120>    Method for Detecting of Nucleic Acid Using
         Intercalator-Conjugated Metal Nanoparticle

<130>    P11-B052

<160>    2

<170>    KopatentIn 1.71

<210>    1
<211>    18
<212>    DNA
<213>    Artificial Sequence

<220>
<223>    Target DNA


<400>    1
tgagcagaat aaaccatt                                                      18


<210>    2
<211>    17
<212>    DNA
<213>    Artificial Sequence

<220>
<223>    Control


<400>    2
gatggctgct tgatgtc                                                       17

## Description

### [Technical Field]

The present invention relates to a method of detecting nucleic acids using metal nanoparticles conjugated with an intercalator, and more specifically, to a method in which a sample containing target nucleic acids is applied to a DNA chip in which probes are immobilized on a substrate, metal nanoparticles conjugated with an intercalator are reacted, a metal enhancing solution is reacted, a size of the metal nanoparticles is amplified, and the target nucleic acids are detected with the naked eye.

### [Background Art]

Biochips refer to biological information detecting elements in which biomaterials such as DNA, proteins, antibodies, sugar chains, cells or neurons are integrated in a high density on a solid substance such as a glass, a silicone, or a polymer, an infinitesimal sample is analyzed at ultra-high speed, biological information such as gene expression patterns, genetic defects, protein distribution, and mutual information exchange between neurons is obtained, and biochemical identification, a reaction rate, or an information processing rate improves.

The biochips may be classified as a DNA chip, an RNA chip, a protein chip, a cell chip, a neuron chip, or the like according to a systematic degree with biomolecules, and may be broadly defined to include "biosensors" that can detect and analyze various biochemical materials such as a lab on a chip that integrates sample pretreatment, biochemical reactions, detection, and data analysis in a small size and has an automatic analyzing function.

In particular, according to progress of the human genome project, DNA chip technology has entered the spotlight as technology for replacing existing molecular biological research methods. In a DNA chip, several tens to several millions of types of DNA fragments are integrated into a very small surface such as a glass slide. A DNA detection method using a DNA chip may detect RNA or DNA contained in a sample of a small amount in a short time, and has been spotlighted because it enables existing Southern blot and Northern blot to be performed on a large scale in a short time. A DNA chip may be applied to mutation detection of genome DNA, gene diagnosis, pharmacogenomics, personalized medicine, and large-scale RNA expression measurement essential for genome research and molecular biological research.

Currently, two types of DNA chips, an oligochip and a cDNA chip, have been introduced. In an oligochip, hundreds of thousands of oligonucleotides of 20 to 25 mers are integrated. In a cDNA chip, cDNA fragments which are longer than the oligonucleotide are integrated.

In a known fundamental principle of a DNA chip, probe DNA fragments having a specific sequence are integrated into a surface called a chip using various methods, and a large amount of probe DNA that bind to target DNA (or RNA) contained in the sample is detected from integrated probe DNA fragments.

Technology for manufacturing and using a DNA chip includes technology for immobilizing a probe that can specifically react with target DNA, technology for detecting whether reactions occur, and information processing technology that can process detected information.

The technology for detecting whether reactions occur generally uses a specific type of label such as fluorescence, color development, and isotopes. Labeling technology is important to increase sensitivity, but biomolecules may be deformed due to labeling and it is difficult to label low molecular substances. In addition, large amounts of samples are used in a labeling procedure, and 2 to 3 operations are further required. A representative labeling method that is currently mainly used includes a fluorescence detection method using a laser. In the fluorescence detection method using a laser, a fluorescent substance is bound to a sample, and reactions with probes immobilized on a substrate are optically determined using the bound fluorescent substance. Although the fluorescence detection method is currently widely used, an optical measurement instrument is required to determine whether reactions occur, thereby requiring much time and costs. In addition, when this detection method is used, it is difficult to minimize an analysis system based on a DNA chip. In addition, an operation of attaching the fluorescent substance to target molecules in the sample is further required, and it is cumbersome compared to a label-free detection method.

Therefore, measurement technology using the label-free method has been increasingly required in the field of DNA chip technology. One such label-free detection method is an electrochemical detection method. In the electrochemical detection method, reactions are detected using electrochemical reactions of other chemical substances on electrodes in which the probe and the sample are bound. However, this method has a relatively lower measurement capability than the fluorescence detection method.

As described above, existing detection methods have limited efficiency. Therefore, when these methods are applied and performed in biochemical experiments, the experiments always involve problems in that reliability and satisfaction in terms of practical usage are minimized.

Accordingly, the inventors have attempted to address problems of inefficiency and additional expensive instruments required for existing DNA chips. When metal nanoparticles conjugated with an intercalator are bound to target nucleic acids bound with DNA probes and a metal enhancing solution is reacted, the metal nanoparticles are amplified to be observable with the naked eye due to the metal enhancing solution, and thus it is possible to perform label-free detection without an additional instrument. Further, the inventors verified that quantitative analysis of the target nucleic acids can be performed using a general scanner and completed the invention.

### [Disclosure]

### [Technical Problem]

The present invention provides a method of quickly detecting and quantifying nucleic acids with the naked eye without an expensive instrument.

### [Technical Solution]

According to an aspect of the invention, there is provided a method detecting nucleic acids using metal nanoparticles conjugated with an intercalator. The method includes (a) applying a sample containing target nucleic acids to a substrate in which DNA probes to be hybridized with the target nucleic acids are immobilized, and hybridizing the probes and the target nucleic acids; (b) reacting the metal nanoparticles conjugated with the intercalator with double helix nucleic acids hybridized in the operation of (a); (c) reacting a metal enhancing solution with the metal nanoparticles bound to the double helix nucleic acids; and (d) detecting the target nucleic acids by analyzing a color change of reacted spots.

According to another aspect of the invention, there is provided a method quantifying nucleic acids using metal nanoparticles conjugated with an intercalator. The method includes (a) applying a sample containing target nucleic acids to a substrate in which probes to be hybridized with the target nucleic acids are immobilized, and hybridizing the probes and the target nucleic acids; (b) reacting the metal nanoparticles conjugated with the intercalator with double helix nucleic acids hybridized in the operation of (a); (c) reacting a metal enhancing solution with the metal nanoparticles bound to the double helix nucleic acids; and (d) quantifying the target nucleic acids by analyzing a color change of reacted spots.

### [Advantageous Effects]

According to the invention, a method of detecting nucleic acids uses metal nanoparticles conjugated with an intercalator. Therefore, it is possible to detect nucleic acids with the naked eye without any instrument and decrease a detection time and an analysis cost compared to an existing detection method. In addition, it is possible to minimize the device and perform field diagnosis in cattle farms or at home.

### [Description of Drawings]

FIG. 1 schematically illustrates a process of a method of detecting nucleic acids according to the invention.

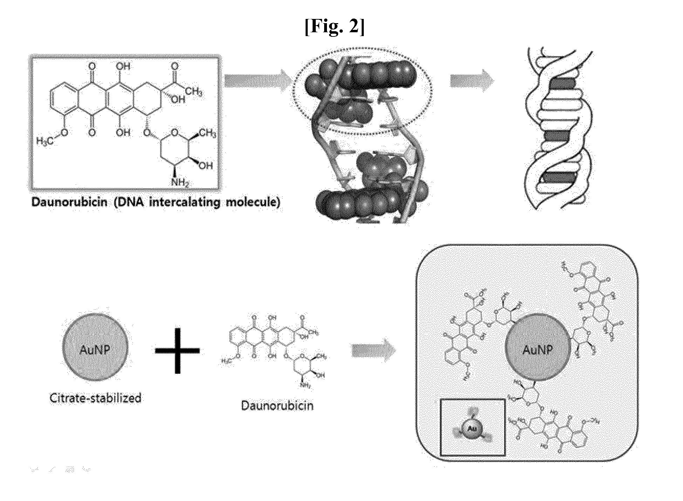

FIG. 2 schematically illustrates a process of binding daunorubicin serving as an intercalator to double helix nucleic acids and a process of conjugating daunorubicin and gold nanoparticles according to the invention.
FIG. 3 is a graph showing spots for each DNA concentration resulting from specific hybridization reactions with target nucleic acid DNA and a quantitative analysis result thereof in a gray scale.

### [Modes of the Invention]

According to an aspect of the invention, there is provided a method of detecting nucleic acids using metal nanoparticles conjugated with an intercalator. The method includes (a) applying a sample containing target nucleic acids to a substrate in which DNA probes to be hybridized with the target nucleic acids are immobilized, and hybridizing the probes and the target nucleic acids; (b) reacting the metal nanoparticles conjugated with the intercalator with double helix nucleic acids hybridized in the operation of (a); (c) reacting a metal enhancing solution with the metal nanoparticles bound to the double helix nucleic acids; and (d) detecting the target nucleic acids by analyzing a color change of reacted spots.

In the invention, a type of the substrate may include a solid substrate for manufacturing a DNA chip that is commonly used in the related art without limitation. Preferably, a glass, alumina, a ceramic, carbon, gold, silver, copper, aluminum, a compound semiconductor, silicone, or the like may be used. More preferably, a glass substrate may be used. Surface treatment is performed on the substrate. The surface treatment is performed to easily attach and immobilize probe molecules. In addition, the surface treatment may be performed to include functional groups for immobilizing biomolecules on a substance surface of the DNA chip. For example, the substrate may be reformed to aldehyde groups, carboxyl groups, or amine groups. When the glass substrate or a semiconductor substrate is used, silane treatment is performed to form amino groups (such as -NH₃ or -NH₂). In order to effectively perform the silane treatment, treatment for creating hydroxyl groups (-OH) may also be performed before the silane treatment. In the invention, any method of immobilizing probe molecules on the substrate may be used without limitation and chemical or physical methods may be used.

In the embodiment of the invention, O₂ plasma treatment was performed on a surface of the glass substrate, -OH groups were exposed to the surface of the glass substrate, the surface was functionalized with amine groups, the surface treated with amine was substituted with carboxyl groups, and DNA serving as the probe having amine groups (-NH₂) was immobilized on the substrate through a peptide bond as a covalent bond. A substrate that was not bound to the DNA was blocked by reacting with polyethylene glycol (PEG) having amine groups to prevent negative staining.

In the invention, the term "DNA probe" refers to a substance that can be specifically bound to target nucleic acids to be detected in the sample and a substance that can specifically check whether target nucleic acids are present in the sample through the binding.

In the invention, the term "target nucleic acid" refers to a substance to be detected in the sample. A type of the target nucleic acid includes DNA, RNA, a peptide nucleic acid (PNA), a locked nucleic acid (LNA), or the like, and more preferably, refers to DNA. More specifically, the target nucleic acid may be derived from an organism as a biomaterial or similarly, or prepared in vitro, DNA includes cDNA, genomic DNA, and oligonucleotides, and RNA includes genomic RNA, mRNA, oligonucleotides, or the like.

In the invention, the term "sample" refers to tissues, cells, whole blood, serum, plasma, saliva, sputum, cerebrospinal fluid, or urine, which contains the target nucleic acid to be detected, but the sample is not limited thereto.

In the invention, the term "spot" refers to a portion in which probes are finely integrated on the substrate, that is, refers to a portion in which DNA probes are immobilized on the substrate such as the DNA chip.

When the sample containing target nucleic acids comes into contact with the substrate having immobilized DNA probes therein, specific binding reactions between probe molecules and target nucleic acids in the sample, that is, specific hybridization reactions between complementary sequences of target nucleic acids and DNA probes, occur.

In the embodiment of the invention, probes capable of detecting target nucleic acids were immobilized on the substrate, and a portion in which probes were not reacted was blocked using blocking molecules in order to reduce non-specific reactions. Here, when the target nucleic acids to be measured are reacted, target nucleic acids and probes, which have complementary sequences, are hybridized and form double helix forms. After the target nucleic acids are reacted, the metal nanoparticles conjugated with the intercalator are reacted, the intercalator is bound to only hybridized double helix nucleic acids and not bound to a non-hybridized portion, and thus nanoparticles are not attached thereto.

In the invention, the term "intercalator" refers to all substances that can be intercalated to double helix nucleic acids and may include streptomycin sulfate, gentamicin sulfate, daunorubicin hydrochloride, nogalamycin, doxorubicin, hedamycin, mitoxantrone, tilorone, hoechst 33258, quinacrine, and acridin orange.

In the embodiment of the invention, a metal nanoparticle solution was prepared, and the intercalator was added and reacted, and then metal nanoparticles conjugated with the intercalator were prepared. Here, a ratio of the intercalator added to the metal nanoparticle solution may be 0.1 mM to 1 mM and a reaction time may be about 3 to 10 hours. In this case, in the conjugated metal nanoparticles, only metal nanoparticles conjugated with daunorubicin were obtained by removing metal nanoparticles that were not bound to the intercalator through an existing refining method such as dialysis.

The metal nanoparticles may include gold (Au), silver (Ag) and platinum (Pt), may be prepared by mixing metal ions and a reducing agent, or may also be easily obtained from a commercial reagent company such as Sigma.

In the invention, the operation of (c) in which the metal enhancing solution is reacted is performed to amplify a size of the metal nanoparticles conjugated with the intercalator bound to the hybridized double helix nucleic acid and thus the target nucleic acids are detected and measured with the naked eye.

In the invention, the term "metal enhancing solution" refers to a solution of metal ions and refers to a solution that can amplify a size of nanoparticles while metal ions around the metal nanoparticles are reduced using the metal nanoparticles as a catalyst. Metal enhancing solutions capable of amplifying a size of nanoparticles that are commonly used in the related art may be used without limitation. Preferably, a solution containing gold (Au), silver (Ag), copper (Cu), platinum (Pt) or palladium (Pd) ions may be used, and more preferably, a solution containing gold ions may be used.

In the invention, the target nucleic acids may be detected by observing a color change of the spot in the operation of (d) with the naked eye, but the invention is not limited thereto. The target nucleic acids may be detected by measuring an intensity change of reflected or transmitted light using optical principles such as a reflection method or a transmission method. In this case, a final detection signal may be represented by a size of a gray scale of black and white.

For example, a gray scale light intensity may be measured using a short wavelength light source such as an LED or a laser diode, and using a photodiode array such as a CMOS or a CCD as a light detecting device, but the invention is not limited thereto. A general optical scanner may be used for analysis.

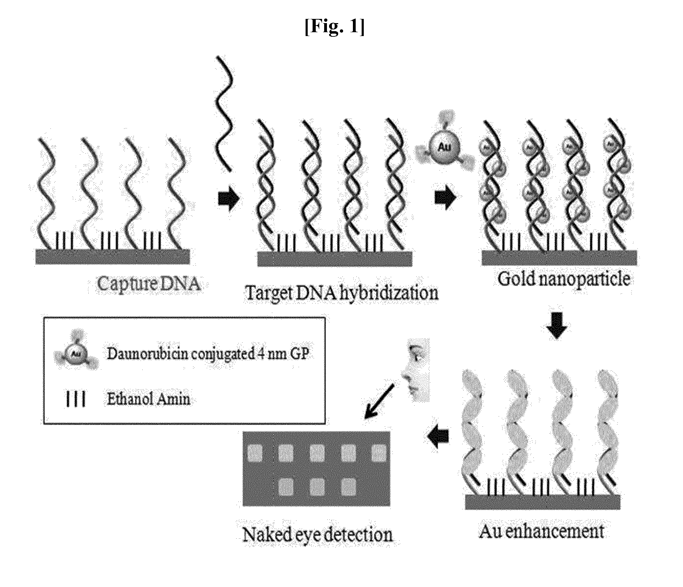

In the embodiment of the invention, gold nanoparticles conjugated with the intercalator were reacted, and a gold enhancing solution was reacted for one minute. As a result, gold ions were reduced, the surroundings of the gold nanoparticles were coated with metals, a size of the particles increased, and a portion to which target nucleic acids were attached appeared in gray and was observed with the naked eye. Specifically, it was observed that a portion that was specifically reacted with probes was expressed in dark gray and a non-specific portion was expressed in a very light gray color or was invisible. Therefore, according to the method of the invention, it is possible to detect the target nucleic acids by simply labeling the fluorescent substance on target nucleic acids or probe molecules, or using the DNA chip with the naked eye without an additional optical instrument or fluorescent scanner (FIG. 1).

According to another aspect of the invention, there is provided a method of quantifying nucleic acids using metal nanoparticles conjugated with an intercalator. The method includes (a) applying a sample containing target nucleic acids to a substrate in which probes to be hybridized with the target nucleic acids are immobilized, and hybridizing the probes and the target nucleic acids, (b) reacting the metal nanoparticles conjugated with the intercalator with double helix nucleic acids hybridized in the operation of (a); (c) reacting a metal enhancing solution with the metal nanoparticles bound to the double helix nucleic acids; and (d) quantifying the target nucleic acids by analyzing a color change of reacted spots.

In the invention, a reaction intensity of a portion that was reacted with the metal enhancing solution in the operation of (d) is measured for quantitative analysis of the target nucleic acids in the sample. As a concentration of the target nucleic acids in the sample increases, the reaction intensity of a portion that was reacted with the metal enhancing solution also increases, and thus it is possible to quantify the target nucleic acids.

A color change of the spot in the operation of (d) may be measured using an intensity change of reflected or transmitted lights using optical principles such as a reflection method or a transmission method. A final detection signal may be represented by a size of a gray scale of black and white.

For example, a gray scale light intensity may be measured using a short wavelength light source such as an LED or a laser diode, and using a photodiode array such as a CMOS or a CCD as a light detecting device, but the invention is not limited thereto. A general optical scanner may be used for analysis.

In the embodiment of the invention, it was observed that a gray spot became darker and larger as a concentration of the target nucleic acids increased from 1 pM to 100 nM. A general scanner was used to capture the change and Adobe Photoshop software was used to analyze the change in a gray scale. As a result, it was observed that, as the concentration of the target nucleic acids increased, a gray scale value also increased, and a surrounding substrate to which the target nucleic acids were not attached showed a constant value regardless of the concentration. Therefore, it is possible to perform quantitative analysis of the target nucleic acids using a general scanner and common software.

Hereinafter, the invention will be described in detail with reference to examples. These examples should be considered in a descriptive sense only and it is apparent to those skilled in the art that the scope of the invention is not limited to the examples.

### Example 1. Method of preparing metal nanoparticles conjugated with intercalator

In order to prepare metal nanoparticles conjugated with an intercalator, 18.5 ml of distilled water was poured into a container. While the container was shaken at 500 RPM, 500 µL of HAuCl₄ (10 mM), 500 µL of Sodium Citrate (10 mM), and 500 µL of NaBH₄ (100 mM) were added and reacted for three hours, 400 µL of 10 % Sodium Dodecyl Sulfate (SDS) was added, and 400 µL of daunorubicin (10 mM, Sigma Aldrich) was added, reacted for six hours or more to reach a final concentration of 0.2 mM, and finally dialyzed in a 0.2% SDS Sodium Citrate (2.5 mM) solution. After daunorubicin that was not attached to gold nanoparticles was removed, gold nanoparticles conjugated with refined daunorubicin were finally obtained.

### Example 2. Treatment of glass substrate

A glass substrate was washed using a piranha solution, O₂ plasma treatment was performed, and -OH groups were exposed to a surface of the glass substrate. The glass substrate was reacted with 2% aminopropyltriethoxysilane (APTES) prepared in an ethanol solution for two hours. After two hours of reaction, the surface was washed with ethanol, dried, and baked on a hot plate of 120 °C for one hour, and thus the surface of the glass substrate was functionalized with amine. The substrate was reacted with succinic anhydride (1M) in dimethylformamide (DMF) overnight and then washed, and the glass substrate treated with amine was substituted with carboxyl groups (-COOH).

### Example 3. Manufacturing of DNA chip

In order to manufacture a DNA chip, first, EDC/NHS was reacted for 15 minutes, 10 µM of DNA consisting of NH₂-O-AATGGTTTATTCTGCTCA (hereinafter referred to as "H5") and 50 µM of control DNA consisting of NH₂-O-GACATCAAGCAGCCATC (hereinafter referred to as "HM") were reacted for one hour, and thus DNA serving as the probe was immobilized in the glass substrate prepared in Example 1. In order to block a portion in which DNA was not immobilized, ethanol amine (1M) having amino-termini was reacted for one hour, and thus the DNA chip having probes attached therein was manufactured.

### Example 4. Specific hybridization reaction with target nucleic acid DNA

In order to check a specific hybridization reaction of target nucleic acid DNA and the DNA chip having probes attached therein prepared in Example 2, in the H5 DNA serving as the probe, target DNA (Bioneer, Korea) having a sequence of TGA GCA GAA TAA ACC ATT, which is complementary sequence ID NO. 1, and a sequence ID NO. 2 of GAT GGC TGC TTG ATG TC serving as the control were diluted with a hybridization buffer (5XSSC, 0.2% SDS), and hybridization reactions were performed for each concentration.

### Example 5. Amplification and measurement of specific hybridization reaction with target nucleic acid DNA

In order to amplify and measure specific hybridization reactions with target nucleic acid DNA, the DNA chip on which hybridization reactions were performed in Example 4 was washed with an SSC buffer solution and non-reacted DNA was removed. Then, gold nanoparticles conjugated with the intercalator were reacted for 10 minutes, washed with the SSC buffer solution, and reacted with the gold enhancing solution (0.85 mL HAuCl₄ (10 mM), 0.25 mL AgNO₃ (10 mM), 0.27 mL Ascorbic acid (100 mM), 20 mL CTAB (100 mM)) for one minute. Then, the DNA chip on which the hybridization reaction was conducted was washed with water, and the specific hybridization reactions were observed.

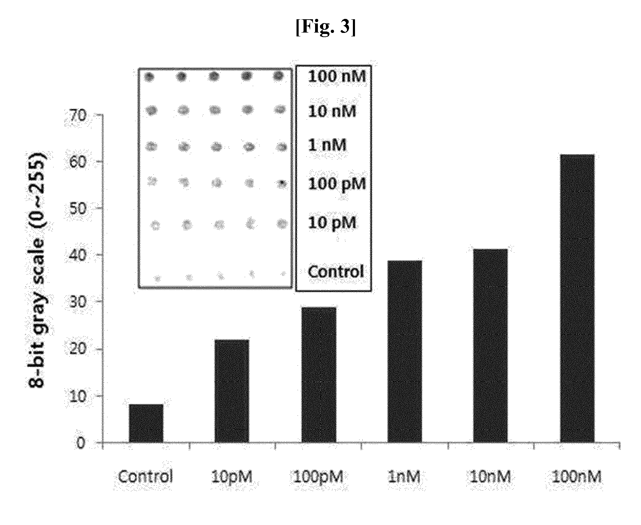

First, spots formed in the glass substrate were observed using a general scanner. As illustrated in FIG. 3, it is possible to observe with the naked eye that gray spots were formed in only portions in which the specific hybridization reactions with target nucleic acid DNA occurred. In FIG. 3, the target nucleic acid DNA was specifically attached to H5 serving as the probe and formed gray spots, and the control DNA was specifically attached to only HN serving as the control and formed gray spots. It is observed that no spot appeared or spots dimly appeared in uncomplimentary probe DNA and target nucleic acid DNA, and a gray color intensity was increased according to the concentration of the target nucleic acid DNA. Therefore, it is verified that the hybridization reaction of the invention was specifically performed, and the intercalator was bound to binding of the target nucleic acid DNA and the probe DNA in which the hybridization reaction occurred. When the gold enhancing solution causing reduction reactions was treated, metal ions were reduced using gold nanoparticles as a catalyst and a size of particles was increased to the extent that spots could be observed with the naked eye without an additional optical instrument.

In addition, Adobe Photoshop software was used to perform quantitative analysis of spots obtained for each concentration of the target nucleic acid DNA in a gray scale. As illustrated in FIG. 3, the result shows that a gray scale value increases from 1 pM to 100 nM for each concentration. On the other hand, it was observed that negative staining was performed on a surrounding substrate to which the target nucleic acid was not attached regardless of the concentration.

The specific parts of content of the invention have been described in detail. However, it will be apparent to those skilled in the art that these specific descriptions are only exemplary examples, and the scope of the invention is not limited thereto. Therefore, the scope of the invention is defined by the accompanying claims and equivalents thereof.

### [Industrial Applicability]

In the method of detecting nucleic acids according to the invention, it is possible to detect nucleic acids with the naked eye without any instrument, minimize the device, and perform field diagnosis.

## Claims

1. A method of detecting nucleic acids using metal nanoparticles conjugated with an intercalator including the following operations, the method comprising:
(a) applying a sample containing target nucleic acids to a substrate in which DNA probes to be hybridized with the target nucleic acids are immobilized, and hybridizing the probes and the target nucleic acids;
(b) reacting the metal nanoparticles conjugated with the intercalator with double helix nucleic acids hybridized in the operation of (a);
(c) reacting a metal enhancing solution with the metal nanoparticles bound to the double helix nucleic acids; and
(d) detecting the target nucleic acids by analyzing a color change of reacted spots.

2. The method of claim 1, wherein the detecting of the target nucleic acids is measured with the naked eye.

3. The method of claim 1, wherein the color change of the spots in the operation of (d) is used to measure an intensity change of reflected or transmitted lights.

4. The method of claim 1, wherein the substrate is selected from the group consisting of a glass, alumina, a ceramic, carbon, gold, silver, copper, aluminum, and silicone.

5. The method of claim 1, wherein the intercalator is selected from the group consisting of streptomycin sulfate, gentamicin sulfate, daunorubicin, nogalamycin, doxorubicin, hedamycin, mitoxantrone, tilorone, hoechst 33258, quinacrine, and acridin orange.

6. The method of claim 1, wherein the metal nanoparticles are selected from the group consisting of gold (Au), silver (Ag), and platinum (Pt).

7. The method of claim 1, wherein the metal enhancing solution is selected from the group consisting of gold solution (Au), silver solution (Ag), copper solution (Cu), platinum solution (Pt), palladium solution, and mixtures thereof.

8. The method of claim 1, wherein the metal enhancing solution in the operation of (c) reduces metal ions and amplifies a size of the metal nanoparticles bound to the double helix nucleic acids.

9. A method of quantifying nucleic acids using metal nanoparticles conjugated with an intercalator including the following operations, the method comprising:
(a) applying a sample containing target nucleic acids to a substrate in which probes to be hybridized with the target nucleic acids are immobilized, and hybridizing the probes and the target nucleic acids;
(b) reacting the metal nanoparticles conjugated with the intercalator with double helix nucleic acids hybridized in the operation of (a);
(c) reacting a metal enhancing solution with the metal nanoparticles bound to the double helix nucleic acids; and
(d) quantifying the target nucleic acids by analyzing a color change of reacted spots.

10. The method of claim 9, wherein the substrate is selected from the group consisting of a glass, alumina, a ceramic, carbon, gold, silver, copper, aluminum, and silicone.

11. The method of claim 9, wherein the intercalator is selected from the group consisting of streptomycin sulfate, gentamicin sulfate, daunorubicin, nogalamycin, doxorubicin, hedamycin, mitoxantrone, tilorone, hoechst 33258, quinacrine, and acridin orange.

12. The method of claim 9, wherein the metal nanoparticles are selected from the group consisting of gold (Au), silver (Ag), and platinum (Pt).

13. The method of claim 9, wherein the metal enhancing solution is selected from the group consisting of gold solution (Au), silver solution (Ag), copper solution (Cu), platinum solution (Pt), palladium solution, and mixtures thereof.

14. The method of claim 9, wherein the metal enhancing solution in the operation of (c) reduces metal ions and amplifies a size of the metal nanoparticles bound to the double helix nucleic acids.

15. The method of claim 9, wherein the color change of the spots in the operation of (d) is used to measure an intensity change of reflected or transmitted lights.
